# EUROPEAN PATENT SPECIFICATION

(11) **EP 4 076 360 B1**
(45) Date of publication and mention of the grant of the patent: **25.03.2026**
(21) Application number: 20838048.5
(22) Date of filing: 18.12.2020
(51) Int. Cl.: A61K 8/34, A61Q 5/06, A61Q 5/12

(54) **COMPOSITION COMPRISING A SOLUBILIZED SOLID FATTY ALCOHOL AND A MONOALCOHOL**
ZUSAMMENSETZUNG, DIE EINEN SOLUBILISIERTEN FESTEN FETTALKOHOL UND EINEN MONOALKOHOL UMFASST
COMPOSITION COMPRENANT UN ALCOOL GRAS SOLIDE SOLUBILISÉ ET UN MONOALCOOL

(30) Priority: 20.12.2019 FR 1915156
(43) Date of publication of application: 26.10.2022
(73) Proprietor: L'OREAL, 75008 Paris (FR)
(72) Inventor: RONCHARD, Guillaume, 93400 SAINT-OUEN (FR); BOUXIROT, Sophie, 93400 SAINT-OUEN (FR)
(74) Representative: L'Oreal
(86) International application number: PCT/EP2020/087227
(87) International publication number: WO 2021/123311

(56) References cited:
- EP-A1- 2 883 533
- EP-B1- 2 883 533
- WO-A1-03/037281
- WO-A1-2015/024079
- WO-A1-2016/090207
- WO-A1-2020/142514
- DE-A1- 102013 215 828
- JP-A- 2002 220 324
- DATABASE GNPD [online] MINTEL; 26 November 2019 (2019-11-26), ANONYMOUS: "Finally! Moisturising Hair Conditioner", XP055720732, retrieved from https://www.gnpd.com/sinatra/recordpage/7059681/ Database accession no. 7059681
- DATABASE GNPD [online] MINTEL; 15 November 2019 (2019-11-15), ANONYMOUS: "Repairing Treatment Mask", XP055720729, retrieved from https://www.gnpd.com/sinatra/recordpage/7038223/ Database accession no. 7038223
- DATABASE GNPD [online] MINTEL; 15 November 2019 (2019-11-15), ANONYMOUS: "Repairing Treatment Mask", XP055720729, retrieved from www.gnpd.com Database accession no. 7038223
- DATABASE GNPD [online] MINTEL; 26 November 2019 (2019-11-26), ANONYMOUS: "Finally! Moisturising Hair Conditioner", XP055720732, retrieved from www.gnpd.com Database accession no. 7059681

## Description

The present invention relates to a composition, notably a cosmetic composition, notably a hair composition, comprising at least 8% by weight of one or more fatty alcohols, at least one of which is a solid fatty alcohol, at least 40% by weight of one or more C₂-C₆ monoalcohols and optionally one or more liquid fatty substances other than liquid fatty alcohols, the composition comprising less than 10% by weight of water and less than 10 % by weight of polyol, relative to the total weight of the composition.

The invention also relates to a process for shaping and/or conditioning keratin fibres, in particular human keratin fibres such as the hair, comprising at least one step of applying to said fibres the composition according to the invention.

The invention lastly relates to the use of the composition according to the invention for shaping and/or conditioning keratin fibres, in particular human keratin fibres such as the hair, and notably for defining the hair curls.

Many individuals are unsatisfied with the appearance of their hair: in particular people who have curly hair may wish to maintain the curls but to obtain better curl definition and better regularity, better movement and less volume and frizziness, even in humid conditions. Furthermore, some individuals find it difficult to control, define and/or style their curly hair.

There is a need to provide compositions that can simplify the styling routine, in particular in the case of people with curly hair.

In particular, there is a need to provide compositions which can maintain hair curls while at the same time giving them good definition and regularity, and which make it possible to obtain good control of the frizziness and volume of the hair, and which withstand humidity, while at the same time providing haircare, which may be persistent on shampooing several times. Prior Art documents that concern hair care compositions comprising fatty alcohols are WO 2020/142514 A1, WO 2015/024079 A1 and EP 2 883 533 A1.

In point of fact, the applicant has just discovered, surprisingly, that a composition comprising the combination of one or more solubilized fatty alcohols and at least one C₂-C₄ monoalcohol makes it possible to solve the abovementioned problems.

A subject of the present invention is thus a composition comprising: at least 8.0% by weight, relative to the total weight of the composition, of one or more fatty alcohols, at least one of which is a solid fatty alcohol, at least 40% by weight, relative to the total weight of the composition, of one or more C₂-C₄ monoalcohols and optionally one or more liquid fatty substances other than liquid fatty alcohols, the composition comprising less than 10% by weight of water and less than 10 % by weight of polyol, relative to the total weight of the composition. Advantageously, the fatty alcohol(s) are solubilized in the composition.

The applicant has notably observed that the composition according to the invention makes it possible to obtain well-defined curls, good control of the frizziness and volume of the hair, and to give the hair good manageability. In addition, the composition according to the invention affords a hair conditioning effect and makes it possible in particular to give a pleasant cosmetic feel, notably a smooth, soft, non-tacky feel, to facilitate the disentangling of the hair and to provide sheen. The composition does not introduce a greasy effect and provides volume control.

In addition, the compositions according to the invention are advantageously clear or transparent, hence an original visual effect for hair products of this type.

Furthermore, these compositions can be sprayed.

Furthermore, the composition according to the invention distributes easily on the hair, notably on dry hair, in order to obtain styling care.

A subject of the invention is also a process for shaping and/or conditioning keratin fibres, in particular human keratin fibres such as the hair, comprising at least one step of applying the composition according to the invention to said fibres.

Other subjects, characteristics, aspects and advantages of the invention will emerge even more clearly on reading the description and the example that follows.

In the present description, the expression "at least one" is equivalent to the expression "one or more" and can be substituted for said expression; the expression "between" is equivalent to the expression "ranging from" and can be substituted for said expression, and implies that the limits are included.

The term "keratin fibres" means fibres of human or animal origin, such as head hair, bodily hair, the eyelashes, the eyebrows, wool, angora, cashmere or fur. According to the present invention, the keratin fibres are preferably human keratin fibres, more preferentially the hair.

The composition according to the invention is advantageously in the form of a clear to translucent, more preferentially clear, fluid.

The clarity of the composition according to the invention can be characterized by the measurement of its turbidity, by turbidimetry (in NTU units). In the context of the present invention, the turbidity measurements were carried out with a turbidimeter, model HI 88713-ISO from the company Hanna Instruments.

Preferably, the turbidity of the compositions according to the invention, measured at ambient temperature (25°C) and atmospheric pressure, is less than 400 NTU units, more preferentially between 1 and 250 NTU units, even better still between 3 and 200 NTU units.

### Fatty alcohols

The composition according to the invention comprises at least 8.0% by weight of one or more fatty alcohols relative to the total weight of the composition, at least one of which is solid.

The term "fatty alcohol" means a non-glycerolated and non-oxyalkylenated, linear or branched, saturated or unsaturated aliphatic alcohol comprising from 6 to 40 carbon atoms and comprising at least one hydroxyl group and preferably containing from 8 to 30 carbon atoms, preferably from 12 to 24 carbon atoms, better still from 14 to 22 carbon atoms.

According to the invention, the fatty alcohols are preferably solubilized in the composition.

The term "solubilized fatty alcohol" means that the fatty alcohols do not precipitate from the composition and/or that they do not form crystals. When the composition is checked visually with the naked eye, it is homogeneous, single-phase, and clear or translucent.

The term "liquid" means a compound that is liquid at 25°C and at atmospheric pressure (760 mmHg; i.e. 1.013×10⁵ Pa). Preferably, this compound has a viscosity of less than or equal to 2 Pa.s, preferably less than or equal to 1 Pa.s, more preferentially less than or equal to 0.1 Pa.s measured at a temperature of 25°C and at a shear rate of 1 s⁻¹.

The term "solid" means a compound that is non-liquid at 30°C and at atmospheric pressure (760 mmHg; i.e. 1.013×10⁵ Pa).

### Solid fatty alcohols

The solid (non-liquid) fatty alcohols according to the invention are chosen from saturated or unsaturated, linear or branched alcohols comprising from 8 to 30 carbon atoms, preferably from 12 to 24 carbon atoms, better still from 14 to 22 carbon atoms.

More preferentially, the solid (non-liquid) fatty alcohols according to the invention are chosen from saturated linear alcohols comprising from 8 to 30 carbon atoms, preferably from 12 to 24 carbon atoms, better still from 14 to 22 carbon atoms.

Preferably, the solid fatty alcohols according to the invention are chosen from fatty alcohols having a melting point greater than 30°C, preferentially greater than 35°C.

Preferably, mention may for example be made of cetyl alcohol, stearyl alcohol, behenyl alcohol and myristyl alcohol, and mixtures thereof.

### Liquid fatty alcohols

The liquid fatty alcohols of the invention may be saturated or unsaturated.

The saturated liquid fatty alcohols are preferably branched. They may optionally comprise in their structure at least one aromatic or non-aromatic ring. Preferably, they are acyclic.

More particularly, the saturated liquid fatty alcohols of the invention are chosen from 2-octyldodecanol, isostearyl alcohol, 2-hexyldecanol, 2-butyloctanol and 2-undecylpentadecanol. Octyldodecanol is most particularly preferred.

The liquid unsaturated fatty alcohols contain in their structure at least one double or triple bond.

Preferably, they bear in their structure one or more double bonds. When several double bonds are present, they are preferably 2 or 3 in number and they can be conjugated or non-conjugated.

The unsaturated fatty alcohols can be linear or branched. They may optionally comprise in their structure at least one aromatic or non-aromatic ring. Preferably, they are acyclic.

More particularly, the liquid unsaturated fatty alcohols of the invention are chosen from oleyl alcohol, linoleyl alcohol, linolenyl alcohol and undecylenyl alcohol, and mixtures thereof. Oleyl alcohol is particularly preferred.

In one preferred embodiment, the composition according to the invention comprises at least two distinct fatty alcohols, at least one of which is a solid fatty alcohol.

Preferably, the composition according to the invention comprises at least one solid fatty alcohol and at least one liquid fatty alcohol.

According to this embodiment, the hair conditioning properties are improved, notably a smoother feel, and the hair is softer and more shiny.

Preferably, the ratio of the solid fatty alcohol/total fatty alcohol concentrations by weight is greater than 0.25 and preferably ranges from 0.3 to 1, preferentially from 0.3 to 0.75, more particularly from 0.35 to 0.6.

The composition according to the invention may comprise a total amount of one or more solid fatty alcohols ranging from 1% to 25% by weight, preferably from 2% to 20% by weight, and more preferentially from 3% to 15% by weight, relative to the total weight of the composition.

The composition according to the invention may comprise a total amount of one or more liquid fatty alcohols ranging from 1% to 20% by weight, preferably from 2% to 15% by weight, and more preferentially from 3% to 10% by weight, relative to the total weight of the composition.

The composition according to the invention may comprise a total amount of one or more fatty alcohols ranging from 8% to 25% by weight, preferably from 8% to 20% by weight, and more preferentially from 10% to 15% by weight, relative to the total weight of the composition.

### C₂-C₆ Monoalcohols

The composition according to the present invention comprises one or more C₂-C₆ monoalcohols.

The C₂-C₆ monoalcohols are notably ethanol, isopropanol, tert-butanol, n-butanol. Preferably, the alcohol used is ethanol.

Preferably, the C₂-C₆ alcohol(s) are present notably in a total amount ranging from 50% to 95% by weight, in particular ranging from 60% to 90% by weight, or even from 70% to 90% by weight, relative to the total weight of the composition.

### Liquid fatty substances

The composition according to the present invention preferably comprises one or more liquid fatty substances other than liquid fatty alcohols.

The term "fatty substance" means an organic compound that is insoluble in water at ordinary ambient temperature (25°C) and at atmospheric pressure (760 mmHg, i.e. 1.013×10⁵ Pa), with a solubility in water of less than 5% by weight, preferably less than 1% by weight and even more preferentially less than 0.1% by weight. They generally have in their structure at least one hydrocarbon-based chain including at least 6 carbon atoms. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane. The fatty substances of the invention are not etherified with oxyalkylenated or glycerolated groups. Preferably, they do not contain in their structure a carboxylic acid function COOH.

The term "hydrocarbon" means a compound comprising solely carbon and hydrogen atoms.

The term "fatty acid" means a compound comprising at least one linear or branched, saturated or unsaturated carboxylic acid function, comprising from 6 to 40 carbon atoms.

The term "fatty ester" means an ester of a fatty acid and/or of a fatty alcohol.

The term "fatty ether" means a compound comprising at least one linear or branched, saturated or unsaturated ether function, comprising at least one hydrocarbon-based group containing from 6 to 40 carbon atoms.

The term "non-silicone" means a compound of which the structure does not comprise any silicon atoms, and which therefore notably does not comprise any siloxane groups.

More particularly, the liquid fatty substances are non-silicone substances.

In particular, the fatty substances of the invention are not (poly)oxyalkylenated or (poly)glycerolated ethers.

The term "oil" means a "fatty substance" that is liquid at ambient temperature (25°C) and at atmospheric pressure (760 mmHg or 1.013×10⁵ Pa).

More particularly, the fatty substances are chosen from C₆-C₁₆ liquid hydrocarbons, liquid hydrocarbons comprising more than 16 carbon atoms, non-silicone oils of animal origin, oils of triglyceride type of plant or synthetic origin, fluoro oils, liquid esters of a fatty acid and/or of a fatty alcohol other than triglycerides, liquid fatty ethers, dialkyl carbonates, and mixtures thereof.

It is recalled that the fatty esters and acids more particularly contain at least one saturated or unsaturated, linear or branched hydrocarbon-based group, comprising from 6 to 40 and better still from 8 to 30 carbon atoms, which is optionally substituted, in particular, with one or more hydroxyl groups (in particular 1 to 4). If they are unsaturated, these compounds may comprise one to three conjugated or unconjugated carbon-carbon double bonds.

As regards the C₆-C₁₆ liquid hydrocarbons, they may be linear or branched, optionally cyclic, and are preferably alkanes. By way of example, mention may be made of hexane, cyclohexane, undecane, dodecane, isododecane, tridecane or isoparaffins, such as isohexadecane or isodecane, and mixtures thereof. C₆-C₁₆ isoparaffins are particularly preferred.

The linear or branched hydrocarbons of mineral or synthetic origin comprising more than 16 carbon atoms are preferably chosen from liquid paraffins, petroleum jelly, liquid petroleum jelly, polydecenes and hydrogenated polyisobutene such as Parleam^{®} 4, and mixtures thereof.

The triglyceride oils of plant or synthetic origin are preferably chosen from liquid triglycerides of fatty acids comprising from 6 to 30 carbon atoms, such as heptanoic or octanoic acid triglycerides, or else, for example, sunflower oil, linseed oil, olive oil, safflower oil, argan oil, maize germ oil, wheat germ oil, soybean oil, marrow seed oil, grapeseed oil, sesame oil, hazelnut oil, apricot kernel oil, macadamia oil, arara oil, castor oil, avocado oil, caprylic/capric acid triglycerides, for instance those sold by the company Stéarineries Dubois or those sold under the names Miglyol^{®} 810, 812 and 818 by the company Dynamit Nobel, shea butter, and mixtures thereof.

### Fatty esters

As regards the liquid esters of fatty acids and/or of fatty alcohols other than the triglycerides mentioned above and the fatty esters a) described above, mention may be made especially of esters of saturated or unsaturated, linear C₁-C₂₆ or branched C₃-C₂₆ aliphatic monoacids or polyacids and of saturated or unsaturated, linear C₁-C₂₆ or branched C₃-C₂₆ aliphatic monoalcohols or polyalcohols, the total carbon number of the esters being greater than or equal to 10.

Preferably, for the esters of monoalcohols, one at least of the alcohol or of the acid from which the esters of the invention are derived is branched and/or unsaturated.

Among the monoesters, mention may be made of dihydroabietyl behenate; octyldodecyl behenate; isocetyl behenate; decyl oleate; isocetyl isostearate; isocetyl laurate; isocetyl stearate; isodecyl octanoate; isodecyl oleate; isononyl isononanoate; isostearyl palmitate; methyl acetyl ricinoleate; octyl isononanoate; 2-ethylhexyl isononate; octyldodecyl erucate; oleyl erucate; ethyl palmitate, isopropyl palmitate, 2-ethylhexyl palmitate, 2-octyldecyl palmitate, alkyl myristates such as isopropyl myristate and 2-octyldodecyl myristate, isobutyl stearate; 2-hexyldecyl laurate, and mixtures thereof.

Preferably, among the monoesters of monoacids and of monoalcohols, use will be made of isopropyl palmitates, alkyl myristates such as isopropyl myristate, isocetyl stearate , 2-ethylhexyl isononanoate, isodecyl neopentanoate, isononyl isononanoate, and mixtures thereof. Still within the context of this variant, use may also be made of esters of C₄-C₂₂ dicarboxylic or tricarboxylic acids and of C₁-C₂₂ alcohols, and esters of monocarboxylic, dicarboxylic or tricarboxylic acids and of C₂-C₂₆ dihydroxy, trihydroxy, tetrahydroxy or pentahydroxy alcohols.

Mention may notably be made of: diethyl sebacate; diisopropyl sebacate; diisopropyl adipate; di-n-propyl adipate; dioctyl adipate; dioctyl maleate; glyceryl undecylenate; octyldodecyl stearoyl stearate; pentaerythrityl monoricinoleate; pentaerythrityl tetraisononanoate; pentaerythrityl tetrapelargonate; pentaerythrityl tetraisostearate; pentaerythrityl tetraoctanoate; propylene glycol dicaprylate; propylene glycol dicaprate; tridecyl erucate; triisopropyl citrate; glyceryl trilactate; glyceryl trioctanoate; propylene glycol dioctanoate; neopentyl glycol diheptanoate; diethylene glycol diisononanoate; and mixtures thereof.

The composition may also comprise, as fatty ester, sugar esters and diesters of C₆-C₃₀ and preferably C₁₂-C₂₂ fatty acids. It is recalled that the term "sugar" refers to oxygen-bearing hydrocarbon-based compounds bearing several alcohol functions, with or without aldehyde or ketone functions, and which include at least 4 carbon atoms. These sugars can be monosaccharides, oligosaccharides or polysaccharides.

As suitable sugars, mention may be made for example of sucrose, glucose, galactose, ribose, fucose, maltose, fructose, mannose, arabinose, xylose, lactose, and derivatives thereof, notably alkyl derivatives, such as methyl derivatives, for instance methylglucose.

The esters of sugars and of fatty acids may especially be chosen from the group comprising the esters or mixtures of esters of sugars described previously and of linear or branched, saturated or unsaturated C₆-C₃₀, preferably C₁₂-C₂₂, fatty acids. If they are unsaturated, these compounds may comprise one to three conjugated or unconjugated carbon-carbon double bonds.

The esters according to this variant may also be chosen from mono-, di-, tri- and tetraesters, polyesters, and mixtures thereof.

These esters may be, for example, oleates, laurates, palmitates, myristates, behenates, cocoates, stearates, linoleates, linolenates, caprates and arachidonates, or mixtures thereof such as, notably, oleopalmitate, oleostearate and palmitostearate mixed esters.

More particularly, use is made of monoesters and diesters and notably sucrose, glucose or methylglucose mono- or di- oleates, stearates, behenates, oleopalmitates, linoleates, linolenates and oleostearates, and mixtures thereof.

An example that may be mentioned is the product sold under the name Glucate^{®} DO by the company Amerchol, which is a methylglucose dioleate.

According to one preferred embodiment, use may also be made of dialkyl carbonates, in particular (C₁-C₁₂)dialkyl carbonates, preferably (C₄-C₁₂)dialkyl carbonates such as notably dicaprylyl carbonate.

### Fatty ethers

The liquid fatty ethers may be chosen from dialkyl ethers, in particular (C₁-C₁₂)dialkyl ethers, preferably (C₄-C₁₂)dialkyl ethers, such as notably dicaprylyl carbonate.

According to one embodiment, the liquid fatty substance(s) is (are) chosen from C₆-C₁₆ alkanes, in particular branched C₈-C₁₆ alkanes, fatty ethers, dialkyl carbonates and mixtures thereof.

According to one preferred embodiment, the liquid fatty substance(s) are chosen from branched C₈-C₁₆ alkanes, (C₁-C₁₂)dialkyl ethers, (C₁-C₁₂)dialkyl carbonates and mixtures thereof.

The composition according to the invention can comprise the liquid fatty substance(s) other than liquid fatty alcohols in a total content ranging from 1% to 20% by weight, preferably from 2% to 15% by weight, preferentially from 5% to 12% by weight, relative to the total weight of the composition.

The composition according to the invention may also comprise a thickener. The thickeners are notably chosen from polysaccharides.

The composition according to the invention may be for cosmetic use. For the purposes of the invention, the term "cosmetic" means a composition of pleasant appearance, odour and feel. Preferably, the composition is for topical application to the skin and keratin fibres, and more especially to the scalp, hair and eyelashes, and even better still to the hair.

The composition according to the invention may be in the form of an alcoholic or aqueous-alcoholic solution of liquid or semi-liquid consistency.

When the composition according to the invention is aqueous, the water content of the composition according to the invention is advantageously less than 10% by weight, preferably ranges from 1% to 8% by weight, in particular from 1% to 5% by weight, relative to the total weight of the composition.

Preferably, the composition according to the invention is anhydrous.

For the purposes of the present invention, the term "anhydrous" means that the water content is less than 1% by weight, preferably less than 0.5% by weight, and better still is free of water.

The composition according to the invention may also comprise one or more additional organic solvents, other than C₂-C₆ monoalcohols, for example chosen from polyols, especially those containing from 2 to 6 carbon atoms, for instance glycerol, propylene glycol, butylene glycol, pentylene glycol, hexylene glycol, dipropylene glycol or diethylene glycol; and polyol ethers such as 2-butoxyethanol, propylene glycol monomethyl ether, and diethylene glycol monoethyl ether and monomethyl ether; and mixtures thereof.

The composition according to the invention comprises less than 10% by weight, preferentially less than 5%, more preferentially less than 2% by weight, and better still less than 1% by weight of polyol(s), relative to the total weight of the composition. More particularly, the composition does not comprise any polyol.

The composition according to the invention may comprise one or more surfactants chosen from non-ionic, anionic, amphoteric or cationic surfactants, and mixtures thereof.

The composition according to the invention may also comprise one or more additional compounds chosen from natural or synthetic thickeners or viscosity regulators; UV-screening agents, fillers such as nacres, titanium dioxide, resins and clays, fragrances, peptizers, vitamins, acidic agents, alkaline agents, colorants, nacreous agents, opacifiers, film-forming polymers, fixing polymers, conditioning agents, solid fatty substances other than solid fatty alcohols, antioxidants and preserving agents.

Those skilled in the art will take care to select the optional additional compounds and the amount thereof such that they do not harm the properties of the composition of the present invention, notably the solubilization of the fatty alcohols.

These additives may be present in the composition according to the invention in an amount ranging from 0 to 20% by weight, relative to the total weight of the composition.

When the composition is aqueous, the pH of the composition according to the invention generally ranges from 2 to 12, preferably from 2.5 to 9, preferentially from 3 to 8 and better still from 4 to 7.

The pH of the composition may be adjusted to the desired value by means of basifying agents or acidifying agents that are customarily used. Among the basifying agents, examples that may be mentioned include aqueous ammonia, alkanolamines, and mineral or organic hydroxides. Among the acidifying agents, examples that may be mentioned include mineral or organic acids, for instance hydrochloric acid, orthophosphoric acid or sulfuric acid, carboxylic acids, for instance acetic acid, tartaric acid, citric acid and lactic acid, and sulfonic acids.

The cosmetic composition may thus be rinsed off or left on after having been applied to the keratin materials, for example rinsed off with water, after an optional leave-on time. The composition is preferably a leave-on composition.

Another subject of the present invention is a process for shaping and/or conditioning keratin fibres, in particular human keratin fibres such as the hair, comprising at least one step of applying to said fibres a composition as described previously.

This or these steps of applying the composition according to the invention may optionally be preceded by at least one step of washing said fibres using a shampoo.

Moreover, this or these steps of applying the composition according to the invention may optionally be followed by a step of leaving the composition on, for example a leave-on time of 5 s to 15 minutes, notably 15 s to 5 minutes; then followed by an optional rinsing step, for example with water; and/or a drying step.

Preferably, the keratin fibres are not rinsed after the step(s) of applying the composition according to the invention. The expression "are not rinsed" is intended to mean that no step of rinsing the keratin fibres is carried out for at least 30 minutes, preferentially 1 hour, after the step of applying the composition according to the invention.

A subject of the present invention is also a use of the composition as described above for shaping and/or conditioning keratin fibres, in particular human keratin fibres such as the hair, and notably for defining the hair curls, for improving softness.

Preferably, the composition as described previously is used on curly hair, to define the curls.

The examples that follow serve to illustrate the invention without, however, being limiting in nature.

### Example 1

Compositions 1 and 2 according to the invention and a comparative composition 3 were prepared using the following ingredients, the contents of which are indicated in the tables below (as g of active material).

**[Table 1]**

| Ingredients | 1 (invention) | 2 (invention) | 3 (comparative) |
|---|---|---|---|
| Cetylstearyl alcohol | 11 | 5.5 | |
| Oleyl alcohol | | 5.5 | 11 |
| Ethanol | qs 100 | qs 100 | qs 100 |

The solid fatty alcohols are solubilized in the compositions according to the invention. No fatty alcohol crystals are observed. The compositions are transparent. The compositions are stable.

### Control of the volume and frizziness (anti-frizz)

The control of the volume and frizziness was evaluated on locks of natural Caucasian hair of frizziness type IV, each weighing 2.7 g and measuring 26 cm.

Application of 0.4 g of composition per g of hair, on dry hair.

Trained experts then visually evaluated the control of the volume and frizziness.

**[Table 2]**

| | 1 (invention) | 2 (invention) | 3 |
|---|---|---|---|
| Volume control | +++ | ++ | - |
| Frizziness control | +++ | ++ | - |

Better control of the volume of the hair is observed, in particular there was no frizziness, for the hair treated with the compositions of the invention compared to the comparative composition.

The hair treated with composition 2 was softer and more shiny than the hair treated with compositions 1 and 3.

### Example 2

The composition according to the invention was prepared from the following ingredients, the contents of which are indicated in the tables below (in g of active material).

**[Table 3]**

| Ingredients | 2av (invention) |
|---|---|
| Cetylstearyl alcohol | 5.5 |
| Oleyl alcohol | 5.5 |
| Dicaprylyl ether | 10 |
| Ethanol | qs 100 |

The hair treated with the composition according to the invention exhibits good styling properties (curl definition, control of frizziness), it has a soft and smooth feel. The feel is neither greasy nor loaded.

### Example 3

Compositions A according to the invention and a comparative composition B were prepared using the following ingredients, the contents of which are indicated in the tables below (as g of active material).

**[Table 5]**

| | A invention | B comparative |
|---|---|---|
| Cetylstearyl alcohol | 9 | 7 |
| Dicaprylylether | 10 | 10 |
| Ethanol | QS 100 | Qs 100 |

Compositions A and B were applied on dry curly hair strands (type IV), at a rate of 0,2 g per gr of hair.

Each lock was then suspended vertically in a chamber at controlled humidity (80% RH) for 48 hours before being evaluated by 5 experts.

For each lock, the curl definition and regularity, and softness of the hair, were evaluated in a blind test, by 5 experts, on a scale ranging from 0 (bad) to 5 (very good)

### "Curl definition and regularity" evaluation

The evaluation of the curl definition and regularity were visual: the expert stated whether the curl was well shaped, geometrical, with a regular pitch. A well-defined curl was formed by hair strands that were grouped together.

### "Softness" evaluation

The evaluation of softness was tactile: The experts took the hair strand between the thumb and forefinger and slides his fingers along the hair from the upper part to the tips; it assessed whether the hair was soft, if it did not have any roughness, if the touch was regular.

| | Curl definition | | Curl regularity | | Softness | |
|---|---|---|---|---|---|---|
| | **B** | **A** *invention* | **B** | **A** *invention* | **B** | **A** *invention* |
| Expert 1 | 3 | 4 | 2 | 4 | 1 | 3 |
| Expert 2 | 3 | 4 | 2,5 | 3,5 | 1,5 | 3 |
| Expert 3 | 3 | 3,5 | 2 | 3,5 | 1 | 2,5 |
| Expert 4 | 2,5 | 4 | 2 | 3 | 2 | 3 |
| Expert 5 | 2 | 3,5 | 1 | 2 | 2 | 3 |
| Mean | **2,7** | **3,8** | **1,9** | **3,2** | **1,5** | **2,9** |
| Standard deviation | 0,4 | 0,3 | 0,5 | 0,8 | 0,5 | 0,2 |

The curls treated with composition A according to the invention were better defined and controlled than when treated with comparative composition B. Further the hair treated with composition A according to the invention were softer than when treated with comparative composition B.

Those differences are significantly different according to the standard deviation.

The invention led to a better humidity resistance of softness of the hair and curl definition/control.

## Claims

1. Composition, notably cosmetic composition, comprising:
at least 8.0% by weight, relative to the total weight of the composition, of one or more fatty alcohols, the composition comprising at least one or more solid fatty alcohols,
at least 40% by weight, relative to the total weight of the composition, of one or more C₂-C₆ monoalcohols, and
optionally one or more liquid fatty substances other than liquid fatty alcohols,
the composition comprising less than 10.0% by weight of water relative to the total weight of the composition,
the composition comprising less than 10% by weight of polyol, relative to the total weight of the composition.

2. Composition according to Claim 1, **characterized in that** the solid fatty alcohol(s) are chosen from cetyl alcohol, stearyl alcohol, behenyl alcohol and myristyl alcohol, and mixtures thereof.

3. Composition according to any one of the preceding claims, **characterized in that** it comprises at least one solid fatty alcohol and at least one liquid fatty alcohol.

4. Composition according to either of Claims 1 and 2, **characterized in that** the liquid fatty alcohols are chosen from 2-octyldodecanol, isostearyl alcohol, 2-hexyldecanol, 2-butyloctanol, 2-undecylpentadecanol, oleyl alcohol, linoleyl alcohol, linolenyl alcohol and undecylenyl alcohol, and mixtures thereof.

5. Composition according to any one of the preceding claims, **characterized in that** the total content of the solid fatty alcohol(s) ranges from 1% to 25% by weight, preferably from 2% to 20% by weight and more preferentially from 3% to 15% by weight, relative to the total weight of the composition.

6. Composition according to the preceding claim, **characterized in that** the ratio of the solid fatty alcohol(s)/total fatty alcohol(s) concentrations by weight is greater than 0.25 and preferably ranges from 0.3 to 1, preferentially from 0.3 to 0.75, more particularly from 0.35 to 0.6.

7. Composition according to any one of the preceding claims, **characterized in that** the total content of the fatty alcohol(s) ranges from 8% to 25% by weight, preferably from 8% to 20% by weight and more preferentially from 10% to 15% by weight, relative to the total weight of the composition.

8. Composition according to any one of the preceding claims, **characterized in that** the fatty alcohol(s) are solubilized in said composition.

9. Composition according to any one of the preceding claims, **characterized in that** it comprises one or more C₂-C₆ monoalcohols chosen from ethanol, isopropanol, tert-butanol and n-butanol, preferably ethanol.

10. Composition according to any one of the preceding claims, **characterized in that** it comprises the C₂-C₆ alcohol(s) in a total amount ranging from 50% to 95% by weight, in particular ranging from 60% to 90% by weight, or even from 70% to 90%, relative to the total weight of the composition.

11. Composition according to any one of the preceding claims, **characterized in that** the liquid fatty substance(s) other than the liquid fatty alcohols are chosen from C₆-C₁₆ alkanes, fatty ethers and dialkyl carbonates, and mixtures thereof.

12. Composition according to any one of the preceding claims, **characterized in that** the total content of the liquid fatty substance(s) other than liquid fatty alcohols ranges from 1% to 20% by weight, preferably from 2% to 15% by weight, preferentially from 5% to 12% by weight, relative to the total weight of the composition.

13. Composition according to any one of the preceding claims, **characterized in that** it comprises less than 5% by weight of polyol, relative to the total weight of the composition.

14. Process for shaping and/or conditioning keratin fibres, in particular human keratin fibres such as the hair, comprising at least one step of applying a composition as defined in any one of Claims 1 to 13 to said fibres.

15. Use of the composition as defined according to any one of Claims 1 to 13, for shaping and/or conditioning keratin fibres, in particular human keratin fibres such as the hair, and notably for defining the hair curls, for improving softness.

## Patentansprüche

1. Zusammensetzung, insbesondere kosmetische Zusammensetzung, umfassend:
mindestens 8,0 Gew.-%, bezogen auf das Gesamtgewicht der Zusammensetzung, eines oder mehrerer Fettalkohole, wobei die Zusammensetzung mindestens einen oder mehrere feste Fettalkohole umfasst,
mindestens 40 Gew.-%, bezogen auf das Gesamtgewicht der Zusammensetzung, eines oder mehrerer C₂-C₆-Monoalkohole und
gegebenenfalls eine oder mehrere flüssige Fettsubstanzen, die von flüssigen Fettalkoholen verschieden sind,
wobei die Zusammensetzung weniger als 10,0 Gew.-% Wasser, bezogen auf das Gesamtgewicht der Zusammensetzung, umfasst,
wobei die Zusammensetzung weniger als 10 Gew.-% Polyol, bezogen auf das Gesamtgewicht der Zusammensetzung, umfasst.

2. Zusammensetzung nach Anspruch 1, **dadurch gekennzeichnet, dass** der feste Fettalkohol bzw. die festen Fettalkohole aus Cetylalkohol, Stearylalkohol, Behenylalkohol und Myristylalkohol sowie Mischungen davon ausgewählt ist bzw. sind.

3. Zusammensetzung nach einem der vorhergehenden Ansprüche, **dadurch gekennzeichnet, dass** sie mindestens einen festen Fettalkohol und mindestens einen flüssigen Fettalkohol umfasst.

4. Zusammensetzung nach einem der Ansprüche 1 und 2, **dadurch gekennzeichnet, dass** die flüssigen Fettalkohole aus 2-Octyldodecanol, Isostearylalkohol, 2-Hexyldecanol, 2-Butyloctanol, 2-Undecylpentadecanol, Oleylalkohol, Linoleylalkohol, Linolenylalkohol und Undecylenylalkohol und Mischungen davon ausgewählt sind.

5. Zusammensetzung nach einem der vorhergehenden Ansprüche, **dadurch gekennzeichnet, dass** der Gesamtgehalt des festen Fettalkohols bzw. der festen Fettalkohole im Bereich von 1 bis 25 Gew.-%, vorzugsweise von 2 bis 20 Gew.-% und weiter bevorzugt von 3 bis 15 Gew.-%, bezogen auf das Gesamtgewicht der Zusammensetzung, liegt.

6. Zusammensetzung nach dem vorhergehenden Anspruch, **dadurch gekennzeichnet, dass** das Gewichtsverhältnis der Konzentration des festen Fettalkohols bzw. der festen Fettalkohole zu dem gesamten Fettalkohol bzw. den gesamten Fettalkoholen größer als 0,25 ist und vorzugsweise im Bereich von 0,3 bis 1, vorzugsweise von 0,3 bis 0,75, spezieller von 0,35 bis 0,6, liegt.

7. Zusammensetzung nach einem der vorhergehenden Ansprüche, **dadurch gekennzeichnet, dass** der Gesamtgehalt des Fettalkohols bzw. der Fettalkohole im Bereich von 8 bis 25 Gew.-%, vorzugsweise von 8 bis 20 Gew.-% und weiter bevorzugt von 10 bis 15 Gew.-%, bezogen auf das Gesamtgewicht der Zusammensetzung, liegt.

8. Zusammensetzung nach einem der vorhergehenden Ansprüche, **dadurch gekennzeichnet, dass** der Fettalkohol bzw. die Fettalkohole in der Zusammensetzung solubilisiert ist bzw. sind.

9. Zusammensetzung nach einem der vorhergehenden Ansprüche, **dadurch gekennzeichnet, dass** sie einen oder mehrere C₂-C₆-Monoalkohole, die aus Ethanol, Isopropanol, tert.-Butanol und n-Butanol ausgewählt sind, vorzugsweise Ethanol, umfasst.

10. Zusammensetzung nach einem der vorhergehenden Ansprüche, **dadurch gekennzeichnet, dass** sie den C₂-C₆-Alkohol bzw. die C₂-C₆-Alkohole in einer Gesamtmenge im Bereich von 50 bis 95 Gew.-%, insbesondere im Bereich von 60 bis 90 Gew.-% oder sogar 70 bis 90 Gew.-%, bezogen auf das Gesamtgewicht der Zusammensetzung, umfasst.

11. Zusammensetzung nach einem der vorhergehenden Ansprüche, **dadurch gekennzeichnet, dass** die flüssige Fettsubstanz, die von flüssigen Fettalkoholen verschieden ist, bzw. die flüssigen Fettsubstanzen, die von flüssigen Fettalkoholen verschieden sind, aus C₆-C₁₆-Alkanen, Fettethern und Dialkylcarbonaten und Mischungen davon ausgewählt ist bzw. sind.

12. Zusammensetzung nach einem der vorhergehenden Ansprüche, **dadurch gekennzeichnet, dass** der Gesamtgehalt der flüssigen Fettsubstanz, die von flüssigen Fettalkoholen verschieden ist, bzw. der flüssigen Fettsubstanzen, die von flüssigen Fettalkoholen verschieden sind, im Bereich von 1 bis 20 Gew.-%, vorzugsweise von 2 bis 15 Gew.-%, bevorzugt von 5 bis 12 Gew.-%, bezogen auf das Gesamtgewicht der Zusammensetzung, liegt.

13. Zusammensetzung nach einem der vorhergehenden Ansprüche, **dadurch gekennzeichnet, dass** sie weniger als 5 Gew.-% Polyol, bezogen auf das Gesamtgewicht der Zusammensetzung, umfasst.

14. Verfahren zum Gestalten und/oder Konditionieren von Keratinfasern, insbesondere menschlichen Keratinfasern wie dem Haar, umfassend mindestens einen Schritt des Aufbringens einer Zusammensetzung gemäß einem der Ansprüche 1 bis 13 auf die Fasern.

15. Verwendung der Zusammensetzung gemäß einem der Ansprüche 1 bis 13 zum Gestalten und/oder Konditionieren von Keratinfasern, insbesondere menschlichen Keratinfasern wie dem Haar, und insbesondere zum Definieren der Haarlocken, zum Verbessern der Weichheit.

## Revendications

1. Composition, notamment composition cosmétique, comprenant :
au moins 8,0 % en poids, par rapport au poids total de la composition, d'un ou plusieurs alcools gras, la composition comprenant au moins un ou plusieurs alcools gras solides,
au moins 40 % en poids, par rapport au poids total de la composition, d'un ou plusieurs monoalcools en C₂-C₆, et éventuellement une ou plusieurs substances grasses liquides différentes des alcools gras liquides,
la composition comprenant moins de 10,0% en poids d'eau par rapport au poids total de la composition,
la composition comprenant moins de 10 % en poids de polyol, par rapport au poids total de la composition.

2. Composition selon la revendication 1, **caractérisée en ce que** le ou les alcools gras solides sont choisis parmi l'alcool cétylique, l'alcool stéarylique, l'alcool béhénylique, l'alcool myristique et leurs mélanges.

3. Composition selon l'une quelconque des revendications précédentes, **caractérisée en ce qu'**elle comprend au moins un alcool gras solide et au moins un alcool gras liquide.

4. Composition selon l'une ou l'autre des revendications 1 ou 2, **caractérisée en ce que** les alcools gras liquides sont choisis parmi le 2-octyldodécanol, l'alcool isostéarylique, le 2-hexyldécanol, le 2-butyloctanol, le 2-undécylpentadécanol, l'alcool oléique, l'alcool linoléique, l'alcool linolénique ou l'alcool undécylénique et leurs mélanges.

5. Composition selon l'une quelconque des revendications précédentes, **caractérisée en ce que** la teneur totale de l'alcool ou des alcools gras solides se situe dans la plage de 1 % à 25 % en poids, de préférence de 2 % à 20 % en poids, et plus préférentiellement de 3 % à 15 % en poids, par rapport au poids total de la composition.

6. Composition selon la revendication précédente, **caractérisée en ce que** le rapport des concentrations en poids alcool(s) gras solide(s) / alcool(s) gras total(s) est supérieur à 0,25 et de préférence va de 0,3 à 1, préférentiellement de 0,3 à 0,75, plus particulièrement de 0,35 à 0,6.

7. Composition selon l'une quelconque des revendications précédentes, **caractérisée en ce que** la teneur la teneur totale de l'alcool ou des alcools gras se situe dans la plage de 8 % à 25 % en poids, de préférence de 8 % à 20 % en poids, et plus préférentiellement de 10 % à 15 % en poids, par rapport au poids total de la composition.

8. Composition selon l'une quelconque des revendications précédentes, **caractérisée en ce que** l'alcool ou les alcools gras sont solubilisés dans ladite composition.

9. Composition selon l'une quelconque des revendications précédentes, **caractérisée en ce qu'**elle comprend un ou plusieurs monoalcools en C₂-C₆ choisis parmi l'éthanol, l'isopropanol, le tert-butanol et le n-butanol, de préférence l'éthanol.

10. Composition selon l'une quelconque des revendications précédentes, **caractérisée en ce qu'**elle comprend l'alcool ou les alcools en C₂-C₆ en une quantité totale allant de 50 à 95 % en poids, notamment allant de 60 à 90 % en poids, ou même de 70 à 90 %, par rapport au poids total de la composition.

11. Composition selon l'une quelconque des revendications précédentes, **caractérisée en ce que** la ou les substances grasses liquides autres que les alcools gras liquides sont choisies parmi les alcanes en C₆-C₁₆, les éthers gras et les carbonates de dialkyle, et leurs mélanges.

12. Composition selon l'une quelconque des revendications précédentes, **caractérisée en ce que** la teneur totale de la ou des substances grasses liquides différentes des alcools gras liquides va de 1 % à 20 % en poids, de préférence de 2 % à 15 % en poids, préférentiellement de 5 % à 12% en poids, par rapport au poids total de la composition.

13. Composition selon l'une quelconque des revendications précédentes, **caractérisée en ce qu'**elle comprend moins de 5% en poids de polyol, par rapport au poids total de la composition.

14. Procédé de mise en forme et/ou de conditionnement de fibres kératiniques, en particulier de fibres kératiniques humaines telles que les cheveux, comprenant au moins une étape d'application sur lesdites fibres d'une composition telle que définie dans l'une quelconque des revendications 1 à 13.

15. Utilisation de la composition telle que définie dans l'une quelconque des revendications 1 à 13, pour la mise en forme et/ou le conditionnement de fibres kératiniques, en particulier de fibres kératiniques humaines telles que les cheveux, et notamment pour définir les boucles de cheveux, pour améliorer la douceur.
